# FASCICULE DE BREVET EUROPEEN

(11) **EP 2 331 550 B1**
(45) Date de publication et mention de la délivrance du brevet: **13.11.2013**
(21) Numéro de dépôt: 09748390.3
(22) Date de dépôt: 15.09.2009
(51) Int. Cl.: C07D 493/18, C07H 19/01

(54) **VOIE COURTE DE SYNTHESE DU 1,6:2,3-DIANHYDRO-BETA-D-MANNOPYRANOSE**
KURZER SYNTHESEPFAD FÜR 1,6:2,3-DIANHYDRO-BETA-D-MANNOPYRANOSE
SHORT SYNTHETIC PATHWAY FOR 1,6:2,3-DIANHYDRO-BETA-D-MANNOPYRANOSE

(30) Priorité: 16.09.2008 FR 0805063
(43) Date de publication de la demande: 15.06.2011
(73) Titulaire: SANOFI, 75008 Paris (FR)
(72) Inventeur: BOUVENOT, Laurent, F-75013 Paris (FR); CRUCIANI, Paul, F-75013 Paris (FR); LARGEAU, Denis, F-75013 Paris (FR); ROVERA, Jean-Claude, F-75013 Paris (FR)
(74) Mandataire: Tanguy, Yannick
(86) Numéro de dépôt international: PCT/FR2009/051730
(87) Numéro de publication internationale: WO 2010/031954

(56) Documents cités:
- BAILLIEZ V ET AL: "A practical large-scale access to 1,6-anhydro-[beta]-D-hexopyranoses by a solid-supported solvent-free microwave-assisted procedure" SYNTHESIS 2003 DE, no. 7, 2003, pages 1015-1017, XP002526685 ISSN: 0039-7881
- MILJKOVIC D ET AL: "A CONVENIENT ROUTE TO 6 FUNCTIONALIZED DERIVATIVES OF D GLUCAL" CARBOHYDRATE RESEARCH, vol. 193, 1989, pages 275-278, XP002526687 ISSN: 0008-6215

## Description

La présente invention se rapporte à un nouveau procédé de préparation du 1,6:2,3-dianhydro-β-D-mannopyranose, désigné ci-après « époxyde de Cerny » ou « composé (I) » et correspondant à la formule suivante, dans laquelle les traits en gras représentent des liaisons situées au-dessus du cycle pyranosique : 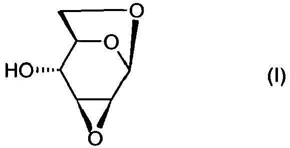 ou encore, selon une autre représentation :

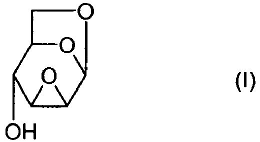

Le composé (I), et plus généralement les composés de la famille des 1,6:(2,3 et 3,4)-dianhydro-β-D-hexopyranoses, ont essentiellement été décrits par un chimiste tchèque, Miloslav Cerny. On trouve dans la littérature trois voies d'accès à l'époxyde de Cerny (I) à partir du composé 1 (1,6:3,4-dianhydro-4-O-tosyl-β-D-galactopyranose):

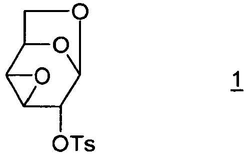

On obtient le composé 1 à partir du lévoglucosane 2 (ou 1,6-anhydro-β-D-glucopyranose), comme représenté ci-dessous (M. Cerny et al., Collect. Czech. Chem. Commun., 1961, vol. 26, p. 2542-2550) :

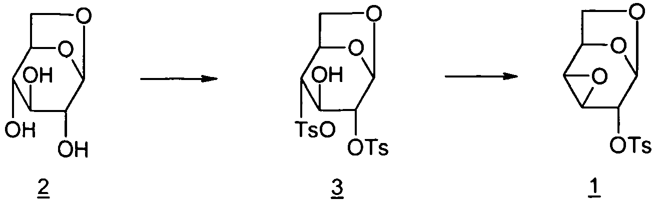

Le dérivé ditosylé 3 (1,6-anhydro-2,4-di-O-tosyl-β-D-glucopyranose) est obtenu sélectivement (80%). Les 20% restant sont essentiellement composés du dérivé tritosylé. Le rendement global pour la transformation du composé 2 en composé 1 est de 55%.

Les voies d'accès au lévoglucosane 2 sont nombreuses ; les plus utilisées industriellement sont, outre la pyrolyse de l'amidon et de la cellulose décrite depuis les années 1960, les cyclisations en milieu basique ou acide du D-glucose, représentées ci-dessous :

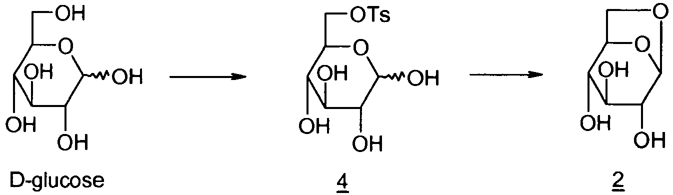

### Cyclisation en milieu basique

### 4 : 6-O-tosyl-D-glucopyranose

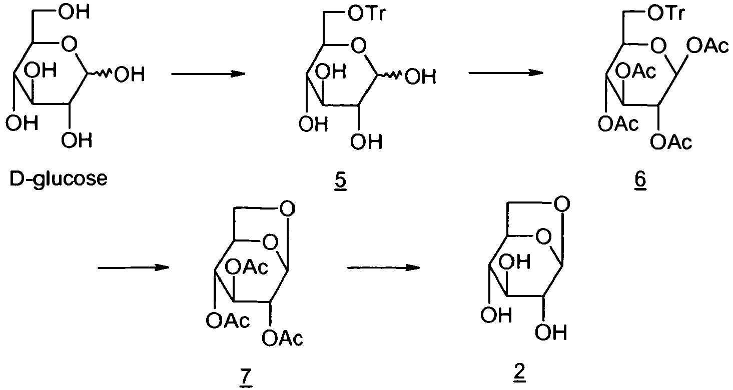

### Cyclisation en milieu acide

### 5 : 6-O-trityl-D-glucopyranose

### 6 : 1,2,3,4-tétra-O-acétyl-6-O-trityl-β-D-glucopyranose

### 7 : 1,6-anhydro-2,3,4-tri-O-acétyl-β-D-glucopyranose

La cyclisation en milieu basique (M.A. Zottola et al., J. Org. Chem., 1989, vol. 54, p. 6123-6125 ; M. Akagi et al., Chem. Pharm. Bull., 1962, vol. 10, p. 905-909) se traduit par un rendement faible (15%). De plus il est nécessaire d'acétyler le lévoglucosane 2 brut pour permettre son isolement. Quant à la voie de cyclisation en milieu acide (M.V. Rao et al., Carbohydrate Research, 1987, vol. 162, 141-144 ; R.L. Wistler et al., Methods Carbohydr. Chem., 1972, vol. 6, p. 411-412 ; E. Zara-Kaczian *et al.,* 1982, vol. 111, no. 3, p. 271-283 ; E. Zara-Kaczian et al., Acta Chemica Acad. Scient. Hung., 1978, vol. 96, no. 3, p. 311-313), elle est décrite avec un meilleur rendement (70%), mais elle comporte deux étapes de plus.

Les trois voies d'accès à l'époxyde de Cerny (I) à partir du composé 1 sont les suivantes.

### Voie 1 :

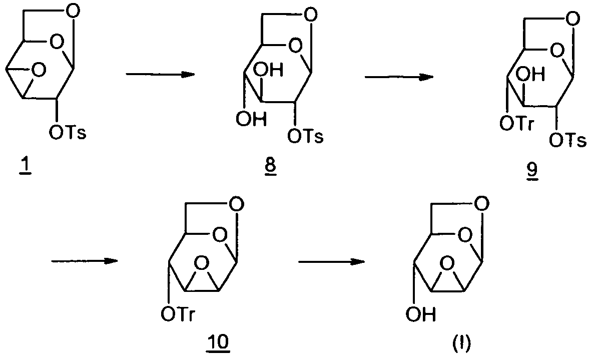

### 8 : 1,6-anhydro-2-O-tosyl-β-D-glucopyranose

### 9 : 1,6-anhydro-2-O-tosyl-4-O-trityl-β-D-glucopyranose

### 10 : 1,6:2,3-dianhydro-4-O-trityl-β-D-glucopyranose

Outre le nombre d'étapes nécessaires pour parvenir à l'époxyde de Cerny (I), cet enchaînement comporte, entre autres, la difficulté d'hydrolyser sélectivement la fonction anhydro-3,4 lors de la première étape. L'hydroxyle en position 4 du dérivé monotosylé 8 est ensuite protégée par un groupement trityle (Tr) afin d'éviter la migration de l'époxyde lors de la cyclisation en présence d'éthylate de sodium (EtONa).

### Voie 2 :

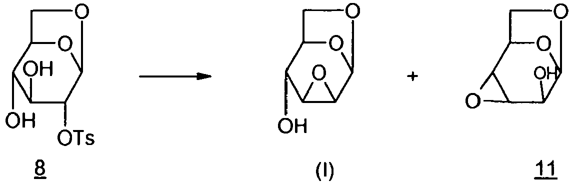

D'après M. Cerny et al. (Synthesis, 1972, 698-699), l'époxyde de Cerny (I) peut être obtenu à partir du dérivé 8 en présence de résine Amberlite IRA 400 / OH⁻. Toutefois, un contact prolongé avec la résine entraîne la migration de l'époxyde en position 3,4 et la formation du dérivé 11 (1,6:3,4-dianhydro-β-D-altropyranose). Il subsiste ainsi la difficulté d'obtenir sélectivement le composé (I). Le composé de départ 8 est lui-aussi difficile à obtenir sélectivement, comme mentionné précédemment.

### Voie 3 :

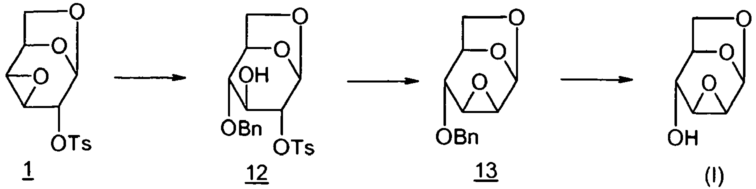

### 12 : 1,6-anhydro-4-O-benzyl-2-O-tosyl-β-D-glucopyranose

### 13 : 1,6:2,3-dianhydro-4-O-benzyl-β-D-mannopyranose

Cette variante permet la cyclisation en dérivé dianhydro 13 sans migration d'époxyde (T. Trnka et al., Collect. Czech. Chem. Commun., 1971, vol. 36, p. 2216-2225 ; M. Cerny et al., Collect. Czech. Chem. Commun., 1968, vol. 33, p. 1143-1156). Elle comporte néanmoins un grand nombre d'étapes pour fournir l'époxyde de Cerny (I) à partir du D-glucose.

En conclusion, les trois voies d'accès décrites ci-dessus pour la préparation de l'époxyde de Cerny (I) comptent respectivement 10, 8 et 9 étapes à partir du D-glucose (en utilisant, pour l'obtention du lévoglucosane 2, la cyclisation en milieu acide, qui est la voie décrite avec le meilleur rendement) et ont pour rendement global, respectivement pour les voies 1, 2 et 3, 0,5%, 10% et 13%.

Par ailleurs, V. Bailliez et al. ont décrit dans Synthesis, 2003, No. 7, 1015-1017 une voie d'accès au 1,6:3,4-dianhydro-β-D-altropyranose, qui s'accompagne d'une formation minoritaire de 1,6:2,3-dianhydro-β-D-mannopyranose en tant que sous-produit. Selon ces auteurs, l'époxyde de Cerny peut être formé à partir d'un précurseur préalablement cyclisé entre les positions 1 et 6, ou bien un précurseur N-1 de l'époxyde de Cerny acétylé en position 4 peut être obtenu, à hauteur de 5%, en plusieurs étapes à partir de 1,3,4-tri-O-acétyl-2,6-di-O-tosyl glucose soumis à de l'alumine, irradiation sous midroondes et per-O-acétylation.

Compte tenu des coûts de main d'oeuvre et des matières premières, et pour une obtention du composé (I) à l'échelle industrielle, il est nécessaire d'envisager une synthèse plus courte et donc plus rentable. Les Inventeurs ont maintenant trouvé une voie d'accès au composé (I) en deux étapes à partir du D-glucose, qui satisfait aux besoins sus-mentionnés.

Le procédé selon l'invention comprend les étapes représentées ci-dessous dans le schéma 1.

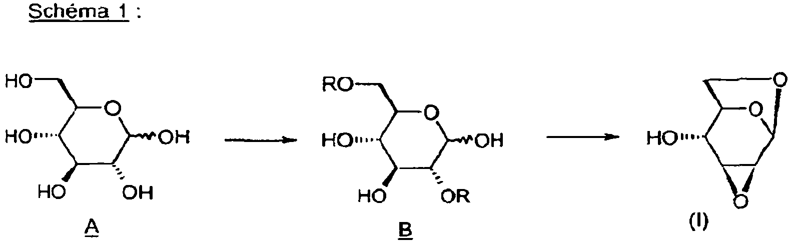

L'invention a ainsi pour objet un procédé de préparation du composé (I), caractérisé en ce qu'il comprend une étape de cyclisation du composé B, dans lequel R représente un groupe activant, en présence d'une base.

Par « agent activant », on entend un agent permettant le départ du groupe partant -OR et favorisant la réaction de cyclisation entre les positions 1 et 6 du composé B, par exemple un halogénure de tosyle, de mésyle, de benzènesulfonyle ou dérivé, tel qu'un halogénure de p-halogéno-benzyle sulfonyle. Le composé B est donc tel que R représente un groupe tosyle, mésyle, benzènesulfonyle ou p-halogéno-benzyle sulfonyle.

Dans le cas du chlorure de losyle (TsCl), le groupe -OTs est un excellent groupe partant. On utilise ainsi avantageusement du chlorure de tosyle en tant qu'agent activant, dans un solvant tel que la pyridine.

La base utilisée lors de l'étape de cyclisation définie précédemment est choisie parmi les hydroxydes d'ammonium et les bases minérales. Les bases minérales utilisables peuvent être des bases minérales fortes (par exemple l'hydroxyde de sodium ou de potassium) ou des bases minérales faibles, notamment de type solide (par exemple le carbonate de potassium, de sodium ou de césium).

On entend par « hydroxyde d'ammonium » un composé de formule N⁺(R₁)(R₂)(R₃)(R₄) OH⁻, dans lequel R₁, R₂, R₃ et R₄, identiques ou différents, représentent des groupes alkyles, lesdits groupes alkyles étant des groupes aliphatiques saturés, linéaires ou ramifiés, comprenant de 1 à 4 atomes de carbone. Un hydroxyde d'ammonium utilisé lors de la réaction de cyclisation du composé B peut par exemple consister en de l'hydroxyde de tétrabutylammonium.

L'étape de cyclisation du composé B est réalisée dans un solvant approprié, choisi en fonction de la nature de la base utilisée, en fonction des connaissances de l'Homme de l'art en ce domaine. La réaction peut par exemple être mise en oeuvre dans de l'isopropanol, du dichlorométhane ou de l'acétonitrile, ou bien dans un mélange binaire de ces solvants.

On utilise par exemple en tant que solvant un mélange isopropanol/dichlorométhane, comprenant par exemple environ 5% en volume de dichlorométhane, lorsque la base utilisée est l'hydroxyde de tétrabutylammonium. Dans ce cas, la réaction de cyclisation est avantageusement réalisée à basse température, notamment à une température inférieure ou égale à 0°C, la présence de dichlorométhane permettant de solubiliser le composé B à basse température. On peut par exemple effectuer la réaction de cyclisation à une température comprise entre -10°C et 0°C, par exemple à environ -5°C.

Lorsque la base utilisée lors de la réaction de cyclisation est le carbonate de césium, on utilise avantageusement l'acétonitrile en tant que solvant, à une température d'environ 40°C.

Comme connu de l'Homme de l'art, la température du milieu réactionnel lors de l'étape de cyclisation est adaptée en fonction du couple solvant/base utilisé, de façon à optimiser la cinétique de réaction.

Le composé (I) est obtenu, selon le procédé de l'invention, avec une sélectivité de 65 à 85% à partir de l'intermédiaire B. Le rendement chimique de cette étape, calculé sur produit isolé, est d'au moins environ 60%.

L'invention a également pour objet un procédé de préparation du composé (I), caractérisé en ce qu'il comprend une étape d'activation du composé A (D-glucose), permettant d'obtenir le composé B, puis une étape de cyclisation du composé B en présence d'une base, telle que définie précédemment.

L'étape d'activation du composé A peut être réalisée à l'aide d'un agent activant tel que défini précédemment. Cette étape est avantageusement réalisée à l'aide d'un halogénure de tosyle, de mésyle, de benzènesulfonyle ou dérivé, tel qu'un halogénure de p-halogéno-benzyle sulfonyle, dans un solvant tel que la pyridine.

L'invention est illustrée à l'aide des exemples qui suivent, qui détaillent un procédé de préparation du composé (I) conformément à l'invention, suivant le schéma 2 ci-dessous.

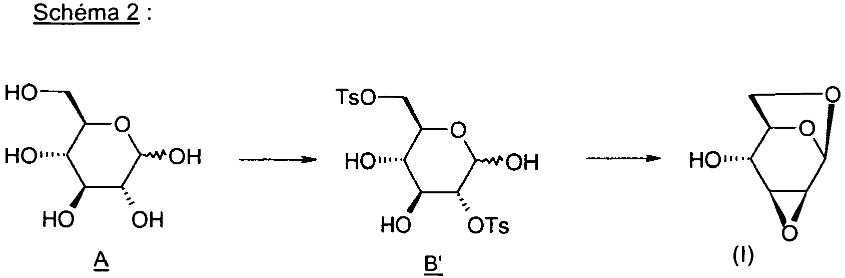

### 1) Préparation du composé B' (2,6-di-O-tosyl-glucopyranose)

Dans un réacteur de 2 litres équipé d'un système d'agitation, on charge 100 g (0,55 mol) de D-glucose et 500 g de pyridine. On refroidit le milieu réactionnel à -10°C. Dans un autre réacteur de 1 litre, on prépare la solution de chlorure de tosyle : on introduit 212 g (1,12 mol) de chlorure de tosyle et 667 g de pyridine et on agite à 20°C jusqu'à dissolution complète. On transfère progressivement (en 4 à 5h) le contenu du réacteur d'1 litre dans le réacteur de 2 litres, tout en maintenant la température à -10°C. On rince avec 39 g de pyridine et on maintient le milieu réactionnel sous agitation pendant 17h à -11°C.

On effectue un échange de solvant par distillation, à l'aide d'eau déminéralisée. La pyridine dans le milieu réactionnel doit être ≤ 20% ; si ce n'est pas le cas on effectue à nouveau une distillation avec 400 ml d'eau.

On refroidit le milieu réactionnel à 20°C et on introduit 400 ml d'eau déminéralisée. Afin d'éliminer le composé monotosylé, on introduit 400 ml de dichlorométhane, 48 g d'acide chlorhydrique et 33 ml d'eau. On laisse reposer 30 min. et on mesure le pH, qui doit être inférieur ou égal à 1 ; si tel n'est pas le cas on rajoute en goutte à goutte de l'acide chlorhydrique jusqu'à l'obtention d'un pH ≤ 1. Puis on effectue des lavages avec une solution de chlorure de sodium (400 ml d'eau + 40 g de chlorure de sodium) jusqu'à l'obtention d'un pH d'environ 5 à 5,5.

Enfin, on concentre au rotavapor la phase dichlorométhane. On reprend le concentrat avec 150 ml de dichlorométhane et on concentre de nouveau. Après avoir effectué trois fois cette opération, on obtient le composé B' sous la forme d'un meringue beige (crème).
Masse de produit attendue = 271 g,
Masse de produit obtenue = 184 g,
Pureté organique = 81.6%, mesurée par HPLC (High Performance Liquid Chromatography),
Rendement chimique = 55%.

### 2) Préparation du composé (I)

### 2.1 : Cyclisation réalisée à l'aide d'hydroxyde de tétrabutylammonium

Dans un réacteur de 1 litre, on charge 10 g de composé B' tel qu'obtenu à l'issue de l'étape précédente, 100 ml d'isopropanol et 5 ml de dichlorométhane. On refroidit le milieu réactionnel à la température à -5°C sous agitation à 400 tr/min. On coule lentement (pendant environ 30 min.) 26,6 g d'hydroxyde de tétrabutylammonium à 40% dans l'eau. On laisse sous agitation pendant 30 minutes et on stoppe la réaction en neutralisant le milieu réactionnel avec de l'acide chlorhydrique à 12%, jusqu'à l'obtention d'un pH d'environ 6 à 7. On change le solvant (remplacement de l'isopropanol par de l'acétate d'éthyle) au rotavapor avec 6 ml d'acétate d'éthyle. Le rendement chimique estimé de cette étape est d'environ 60%, la pureté organique du composé (I) étant de 68%, mesurée selon l'aire des pics en chromatographie en phase gazeuse.

Les spectres RMN du proton et du carbone 13 du composé (I) sont enregistrés sur un appareil Bruker 300 MHz. Les déplacements chimiques sont exprimés par rapport au tétraméthylsilane, à 0,01 ppm près pour le spectre du proton et à 0,1 ppm près pour le spectre du carbone 13. Les constantes de couplage sont données en valeur absolue en Hz à 0,5 Hz près.

RMN ¹H (CDCl₃) : 2,67 (d, 1 H, OH, J _{4,OH} 5,5 Hz), 3,12 (d, 1 H, H₃, J _{2,3} 3,4 Hz), 3,42 (dd, 1 H, H₂, J _{2,3} = J _{2,1} = 3,0 Hz), 3,69 à 3,77 (m, 2H, H₆, H_{6'}), 3,89 (d, 1 H, H₄, J _{4,OH} 5,5 Hz), 4,40 (dm, 1 H, H₁, J _{1,2} 3,0 Hz).

RMN ¹³C: 49,3: C₃; 54,3: C₂; 65,6: C_{6,6'}; 67,1: C₄; 97,7: C₁; 74,2: C₅.

### 2.2 : Cyclisation réalisée à l'aide de carbonate de césium

En variante, on procède comme indiqué dans l'exemple 2.1, mais en utilisant le carbonate de césium en tant que base pour la réaction de cyclisation du composé B'. On utilise 2 équivalents de carbonate de césium par rapport à la quantité de composé B', à savoir 1,133 g de carbonate de césium pour 1 g de composé B', dans 10,5 ml d'acétonitrile et à une température d'environ 40°C. Le rendement chimique estimé de cette étape est d'environ 80%, la pureté organique du composé (I) ainsi obtenu étant de 87%, mesurée selon l'aire des pics en chromatographie en phase gazeuse.

## Revendications

1. Procédé de préparation du composé (I) : 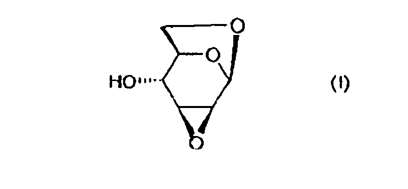 **caractérisé en ce qu'**il comprend une étape de cyclisation du composé B : 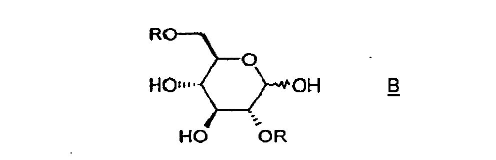 dans lequel R représente un groupe activant, en présence d'une base choisie parmi les hydroxydes d'ammonium et les bases minérales.

2. Procédé selon la revendication 1, **caractérisé en ce que** la base est choisie parmi un hydroxyde d'ammonium, l'hydroxyde de sodium ou de potassium et le carbonate de potassium, de sodium ou de césium.

3. Procédé selon la revendication 1 ou la revendication 2, **caractérisé en ce que** la base est l'hydroxyde de tétrabutylammonlum.

4. Procédé selon la revendication 1 ou la revendication 2, **caractérisé en ce que** la base est le carbonate de césium.

5. Procédé selon l'une quelconque des revendications 1 à 4, **caractérisé en ce que** l'étape de cyclisation est réalisée dans de l'isopropanol, du dichlorométhane ou de l'acétonitrile, ou bien dans un mélange binaire de ces solvants.

6. Procédé selon la revendication 3, **caractérisé en ce que** l'étape de cyclisation est réalisée dans un mélange isopropanol/dichlorométhane.

7. Procédé selon la revendication 6, **caractérisé en ce que** l'étape de cyclisation est réalisée à une température comprise entre -10°C et 0°C.

8. Procédé selon la revendication 4, **caractérisé en ce que** l'étape de cyclisation est réalisée dans l'acétonitrile.

9. Procédé selon la revendication 8, **caractérisé en ce que** l'étape de cyclisation est réalisée à une température d'environ 40°C.

10. Procédé selon l'une quelconque des revendications précédentes, **caractérisé en ce que** le composé B est tel que R représente un groupe tosyle, mésyle, benzènesulfonyle ou p-halogéno-benzyle sulfonyle.

11. Procédé selon l'une quelconque des revendications précédentes, **caractérisé en ce qu'**il comprend :
- une étape d'activation du composé A : 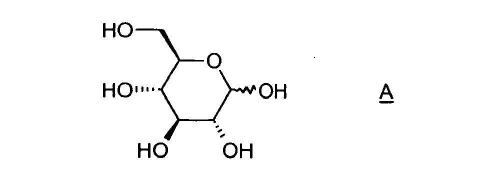 permettant d'obtenir le composé B tel que défini dans la revendication 1,
- suivie d'une étape de cyclisation du composé B, telle que définie dans l'une quelconque des revendications 1 à 9.

12. Procédé selon la revendication 11, **caractérisé en ce que** l'étape d'activation du composé A est réalisée à l'aide d'un halogénure de tosyle, de mésyle, de benzènesulfonyle ou de p-halogéno-benzyle sulfonyle.

## Patentansprüche

1. Verfahren zur Herstellung von Verbindung (I): 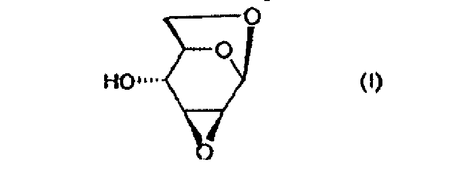 **dadurch gekennzeichnet, dass** man Verbindung B: 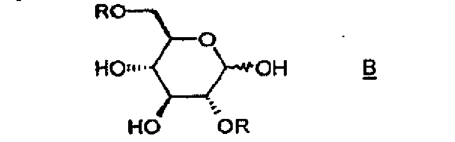 worin R für eine Aktivierungsgruppe steht, in Gegenwart einer aus Ammoniumhydroxiden und anorganischen Basen ausgewählten Base cyclisiert.

2. Verfahren nach Anspruch 1, **dadurch gekennzeichnet, dass** man die Base aus einem Ammoniumhydroxid, Natrium- oder Kaliumhydroxid und Kalium-, Natrium- oder Caesiumcarbonat auswählt.

3. Verfahren nach Anspruch 1 oder Anspruch 2, **dadurch gekennzeichnet, dass** es sich bei der Base um Tetrabutylammoniumhydroxid handelt.

4. Verfahren nach Anspruch 1 oder Anspruch 2, **dadurch gekennzeichnet, dass** es sich bei der Base um Caesiumcarbonat handelt.

5. Verfahren nach einem der Ansprüche 1 bis 4, **dadurch gekennzeichnet, dass** man die Cyclisierung in Isopropanol, Dichlormethan oder Acetonitril oder auch in einer binären Mischung dieser Lösungsmittel durchführt.

6. Verfahren nach Anspruch 3, **dadurch gekennzeichnet, dass** man die Cyclisierung in einer Mischung von Isopropanol und Dichlormethan durchführt.

7. Verfahren nach Anspruch 6, **dadurch gekennzeichnet, dass** man die Cyclisierung bei einer Temperatur zwischen -10°C und 0°C durchführt.

8. Verfahren nach Anspruch 4, **dadurch gekennzeichnet, dass** man die Cyclisierung in Acetonitril durchführt.

9. Verfahren nach Anspruch 8, **dadurch gekennzeichnet, dass** man die Cyclisierung bei einer Temperatur von ungefähr 40°C durchführt.

10. Verfahren nach einem der vorhergehenden Ansprüche, **dadurch gekennzeichnet, dass** die Verbindung B so beschaffen ist, dass R für eine Tosyl-, Mesyl-, Benzolsulfonyl- oder p-Halogenbenzylsulfonylgruppe steht.

11. Verfahren nach einem der vorhergehenden Ansprüche, **dadurch gekennzeichnet, dass** man
- die Verbindung A: 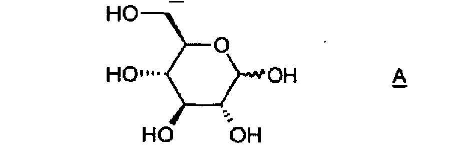 aktiviert, wodurch die Verbindung B gemäß Anspruch 1 erhältlich ist,
- und dann die Verbindung B gemäß einem der Ansprüche 1 bis 9 cyclisiert.

12. Verfahren nach Anspruch 11, **dadurch gekennzeichnet, dass** man die Aktivierung der Verbindung A mit Hilfe eines Tosyl-, Mesyl-, Benzolsulfonyl- oder p-Halogenbenzylsulfonylhalogenids durchführt.

## Claims

1. Process for the preparation of compound (I): 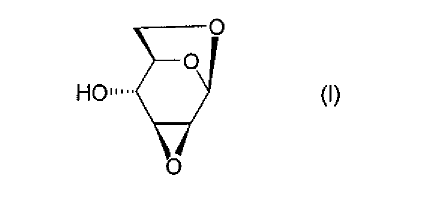 **characterized in that** it comprises a stage of cyclisation of compound B: 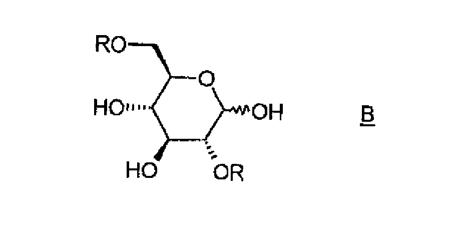 in which R represents an activating group, in the presence of a base chosen from ammonium hydroxides and inorganic bases.

2. Process according to Claim 1, **characterized in that** the base is chosen from an ammonium hydroxide, sodium hydroxide, potassium hydroxide, potassium carbonate, sodium carbonate or caesium carbonate.

3. Process according to Claim 1 or Claim 2, **characterized in that** the base is tetrabutylammonium hydroxide.

4. Process according to Claim 1 or Claim 2, **characterized in that** the base is caesium carbonate.

5. Process according to any one of Claims 1 to 4, **characterized in that** the cyclisation stage is carried out in isopropanol, dichloromethane or acetonitrile, or else in a binary mixture of these solvents.

6. Process according to Claim 3, **characterized in that** the cyclisation stage is carried out in an isopropanol/dichloromethane mixture.

7. Process according to Claim 6, **characterized in that** the cyclisation stage is carried out at a temperature of between -10°C and 0°C.

8. Process according to Claim 4, **characterized in that** the cyclisation stage is carried out in acetonitrile.

9. Process according to Claim 8, **characterized in that** the cyclisation stage is carried out at a temperature of approximately 40°C.

10. Process according to any one of the preceding claims, **characterized in that** compound B is such that R represents a tosyl, mesyl, benzenesulphonyl or p-halobenzenesulphonyl group.

11. Process according to any one of the preceding claims, **characterized in that** it comprises:
- a stage of activation of compound A: 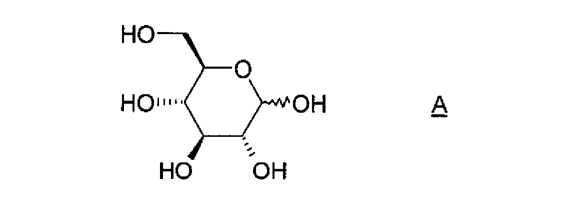 making it possible to obtain compound B as defined in Claim 1,
- followed by a stage of cyclisation, as defined in any one of Claims 1 to 9, of compound B.

12. Process according to Claim 11, **characterized in that** the stage of activation of compound A is carried out using a tosyl, mesyl, benzenesulphonyl or p-halobenzenesulphonyl halide.
